# EUROPEAN PATENT APPLICATION

(11) **EP 2 602 618 A1**
(43) Date of publication of application: **12.06.2013**
(21) Application number: 11814331.2
(22) Date of filing: 28.01.2011
(51) Int. Cl.: G01N 33/50, A61K 36/23, A61K 36/28, A61K 36/53, A61K 45/00, A61P 29/00, A61P 35/00, G01N 33/15, G01N 33/53

(54) **METHOD FOR SCREENING CHRONIC INFLAMMATION SUPPRESSION AGENT OR CANCER METASTASIS SUPPRESSION AGENT HAVING INHIBITION OF BONDING OF EMMPRIN AND S100A9 AS INDICATOR**

(30) Priority: 02.08.2010 JP 2010174038
(71) Applicant: Shiseido Co., Ltd., Tokyo 104-8010 (JP)
(72) Inventor: HIBINO, Toshihiko, Yokohama-shi Kanagawa 236-8643 (JP); EHAMA, Ritsuko, Yokohama-shi Kanagawa 224-8558 (JP); MOTOYAMA, Akira, Yokohama-shi Kanagawa 224-8558 (JP); YAMADA, Shoko, Yokohama-shi Kanagawa 236-8643 (JP); YAMAMOTO, Mami, Yokohama-shi Kanagawa 236-8643 (JP); SAKAGUCHI, Masakiyo, Okayama-shi Okayama 700-8558 (JP); HUH Nam ho, Okayama-shi Okayama 700-8558 (JP)
(74) Representative: Henkel, Breuer & Partner
(86) International application number: PCT/JP2011/051807
(87) International publication number: WO 2012/017700

(57) **Abstract**

The invention provides a screening method for chronic inflammation inhibitors or cancer metastasis inhibitors wherein, when a candidate substance as a chronic inflammation inhibitor or cancer metastasis inhibitor significantly inhibits binding between EMMPRIN and S100A9 or S100A8/A9, the candidate substance is evaluated as significantly suppressing chronic inflammation or cancer metastasis.

## Description

### [Technical field]

The present invention provides a screening method for chronic inflammation inhibitors or cancer metastasis inhibitors which targets EMMPRIN, a novel receptor for S100A9.

### [Background Art]

S100A8 and S100A9 are known as proteins that are upregulated in hyperproliferation and psoriasis. S100A8 and S100A9 belong to the EF-hand calcium-binding S100 protein family, which is composed of over 20 members (NPL 1: Marenholz I et al., Biochem Biophys Res Commun (2004) 322:1111-1122). Both proteins are secreted by neutrophils, activated monocytes and macrophages and function as cellular chemotactic molecules, and they contribute to a positive feedback loop involved in the recruitment of inflammatory cells (NPL 2: Roth J et al., Trends Immunol (2003) 24:155-158). S100A8- and S100A9-positive bone marrow cells are the first cells to infiltrate regions of inflammation (NPL 3: Odink K et al., Nature (1987) 330:80-82). High serum levels of S100A8 and S100A9 have been observed in numerous human inflammatory conditions including chronic rheumatoid arthritis (NPL 4: Liao H et al., Arthritis Rheum (2004) 50:3792-3803), multiple sclerosis (NPL 5: Bogumil T et al., Neurosci Lett (1998) 247:195-197), Crohn disease (NPL 6: Lugering N, et al., Digestion (1995) 56:406-414) and connective tissue disease (NPL 7: Kuruto R, et al., J Biochem (Tokyo) (1990) 108:650-653). Therefore, S100A8 and S100A9 are believed to play important roles in induction and propagation of inflammation.

Regarding the biological functions performed by S100A8 and 100A9 in epithelial cells, the present inventors have previously shown that formation of a complex of extrinsic S100A8 and S100A9 (S100A8/A9) (alternate name: calprotectin) stimulates normal epidermal keratinocytes (NHEK) to produce inflammatory cytokines, whose expression is promoted in psoriatic lesions, and further that S100A8/A9-inducible cytokines stimulate production and secretion of S100A8 and S100A9 in NHEK (NPL 8: J Cell Biochem. 2007 Nov 28, Epub ahead of print). In addition, it has been found that S100A8/A9 itself augments NHEK proliferation. These results demonstrated that a positive feedback mechanism exists between NHEK proliferation and inflammation, with S100A8/A9 acting as the primary mediator. That is, it was suggested that a spiral feedback loop is formed in which S100A8/A9 induces production of inflammatory cytokines that elicit an inflammatory condition, the inflammation inducing cell proliferation, and the cell proliferation in turn inducing further inflammation, and that this is a cause of persistent dermatitic conditions that involve a chain of proliferation and inflammation, such as atopic dermatitis and psoriasis.

In order to block the negative cycle formation of chronic inflammation caused by S100A8/A9, it is believed necessary to identify the receptors for S100A8 and A9.

### [Citation List]

### [Non-patent literature]

[NPL 1] Biochem Biophys Res Commun (2004) 322:1111-1122
[NPL 2] Trends Immunol (2003) 24:155-158
[NPL 3] Nature (1987) 330:80-82
[NPL 4] Arthritis Rheum (2004) 50:3792-3803
[NPL 5] Neurosci Lett (1998) 247:195-197
[NPL 6] Digestion (1995) 56:406-414
[NPL 7] J Biochem (Tokyo) (1990) 108:650-653
[NPL 8] J Cell Biochem. (2008) 104:453-464
[NPL 9] Nature Cell Biol. (2006) 8(12): 1369-1375
[NPL 10] Hum Genet (2002) 111:310-313
[NPL 11] Morrison TB et al., Biotechniques (1998) 24:954-958, 960, 962

### [DISCLOSURE OF THE INVENTION]

### [Problems to be Solved by the Invention]

The present invention provides a screening method for chronic inflammation inhibitors or cancer metastasis inhibitors wherein the target is a novel receptor for S100A9.

### [Means for Solving the Problems]

The present inventors have confirmed that RAGE (Receptor for Advanced Glycation Endproducts), which is known as a receptor of the S100 protein family, also binds with S100A9. However, the presence of a signaling system through their binding could not be confirmed by neutralizing antibody or siRNA tests.

The present inventors therefore isolated proteins that bind with S100A8 and/or A9 from cultured keratinocytes and subjected them to LC/MS/MS analysis and, as a result of attempting to identify the S100A8/A9-binding proteins, discovered numerous receptor candidates for S100A9. Particular attention was focused on EMMPRIN (Extracellular Matrix MetalloPRoteinase INducer) (alternate names: Basigin or CD147), which is a member of the immunoglobulin superfamily and is a membrane-spanning glycoprotein, and it was found that suppressing expression of EMMPRIN significantly reduces cytokine and matrix metalloprotease induction by S100A9. In addition, the results of immunostaining indicated that S100A9 and EMMPRIN are overexpressed in the epidermis of patients suffering from atopic dermatitis or psoriasis, and in infiltrating melanoma cells.

It was therefore found that EMMPRIN is a receptor of S100A9, and that inhibiting their binding suppresses chronic inflammation as well as cancer metastasis, and the present invention was thereupon completed.

The present invention therefore encompasses the following.
(1) A screening method for chronic inflammation inhibitors or cancer metastasis inhibitors wherein, when a candidate substance as a chronic inflammation inhibitor or cancer metastasis inhibitor significantly inhibits binding between EMMPRIN and S100A9 or S100A8/A9, the candidate substance is evaluated as significantly suppressing chronic inflammation or cancer metastasis.
(2) The method according to (1), which comprises incubating EMMPRIN and S100A9 or S100A8/A9 in the presence of a candidate substance as a chronic inflammation inhibitor or cancer metastasis inhibitor, and selecting a substance that inhibits binding between EMMPRIN and S100A9 or S100A8/A9 as a chronic inflammation inhibitor or cancer metastasis inhibitor.
(3) The method according to (1) or (2), wherein the EMMPRIN is solid phased on a solid support.
(4) The method according to any one of (1) to (3), wherein the inhibition of binding is determined by ELISA.
(5) A chronic inflammation inhibitor or cancer metastasis inhibitor comprising a drug that inhibits binding between EMMPRIN and S100A9.
(6) The chronic inflammation inhibitor or cancer metastasis inhibitor according to (5), wherein the drug comprises one or more plant bodies selected from the group consisting of Japanese mugwort, dong quai and white dead-nettle, or their extracts.
(7) A method of suppressing chronic inflammation or cancer metastasis, which comprises administering a drug that inhibits binding between EMMPRIN and S100A9 to a patient.
(8) The method according to (9), wherein the drug comprises one or more plant bodies selected from the group consisting of Japanese mugwort, dong quai and white dead-nettle, or their extracts.

### [Effect of the Invention]

EMMPRIN induces matrix metalloprotease (MMP) and promotes cancer metastasis. The relationship between EMMPRIN and malignant tumor is well known. As regards S100A8/A9 as well, it is known that preferred sites for metastasis react with cancer cell-produced factors such as VEGF and TNF, secreting S100A8/A9, and eliciting metastasis of cancer cells (NPL 9: Nature Cell Biol. (2006) 8(12): 1369-1375). The present inventors have confirmed, upon stimulating cultured keratinocytes with S100A8/A9, that MMP which contributes to cancer infiltration promotes expression by that stimulation, but that the expression is significantly suppressed after knockdown of EMMPRIN (results not shown here). In the prior art, accelerated MMP expression has been thought to be due to an EMMPRIN autocrine loop, but these results suggest that MMP expression is promoted not with EMMPRIN alone, but also requires S100A9 stimulation.

The present inventors have been the first to discover that EMMPRIN, which contributes to cancer cell metastasis, functions as a receptor for S100A9. In fact, suppression of EMMPRIN expression reduces the accelerated expression of cytokines and MMP due to S100A9 (see Examples). Consequently, the relationship between EMMPRIN and S100A9 may be considered to provide a new interpretation of cancer metastasis and its malignancy. In addition, since EMMPRIN has been expressed in the epidermal upper layer of atopic dermatitis and psoriasis patients and in epidermis with melanoma cells, similar to S100A9, it is expected that both contribute to chronic inflammation including atopic dermatitis, psoriasis and cancer. According to the invention, therefore, it is possible to search for chronic inflammation inhibitors or cancer metastasis inhibitors using inhibition of binding between EMMPRIN and S100A9 as the index.

### [Brief Description of the Drawings]

Fig. 1 shows the primary structure of EMMPRIN.
Fig. 2 shows S100A9 receptor candidate proteins, identified by comprehensive analysis of proteins using multidimensional capillary LC/MS/MS.
Fig. 3 shows the EMMPRIN expression-suppressing effect of EMMPRIN siRNA.
Fig. 4 shows changes in cytokine expression due to suppression of EMMPRIN expression.
Fig. 5 shows changes in MMP expression due to suppression of EMMPRIN expression.
Fig. 6 shows identification of EMMPRIN-binding proteins by Western blotting. The arrow indicates the S100A9 band.
Fig. 7 shows the MMP1-inducing effect of soluble EMMPRIN.
Fig. 8A is an immunostain showing localization of EMMPRIN and S100 protein in human normal skin.
Fig. 8B is an immunostain showing localization of EMMPRIN and S100 protein in a skin model.
Fig. 8C is an immunostain showing localization of EMMPRIN and S100 protein in atopic dermatitic skin.
Fig. 8D shows a comparison of atopic dermatitic and psoriatic skin immunostained using S100A8 antibody, 27E10 and Dapi.
Fig. 8E shows a comparison of atopic dermatitic and psoriatic skin immunostained using S100A9 antibody, 27E10 and Dapi.
Fig. 8F is an immunostain showing localization of S100A9 in melanoma tissue (the upper row being HE staining, the middle row S100A9 antibody staining and the lower row DAPI staining. The left, center and right photographs are for different samples.)
Fig. 8G is an immunostain showing localization of S100A9 in malignant melanoma.
Fig. 8H is an immunostain showing localization of EMMPRIN in melanoma tissue.
Fig. 9 is a confirmation photograph (200x) of interaction between EMMPRIN and S100A9 in atopic skin, by PLA (Proximity Ligation Assay).
Fig. 10 is a confirmation photograph (400x) of interaction between EMMPRIN and S100A9 in atopic skin, by PLA.
Fig. 11 shows screening results for plant extracts that inhibit binding between S100A9 and EMMPRIN.
Fig. 12 shows a comparison of the S100A9-EMMPRIN binding inhibition effects of Japanese mugwort extract, white dead-nettle and dong quai extract.

### [Best Mode for Carrying Out the Invention]

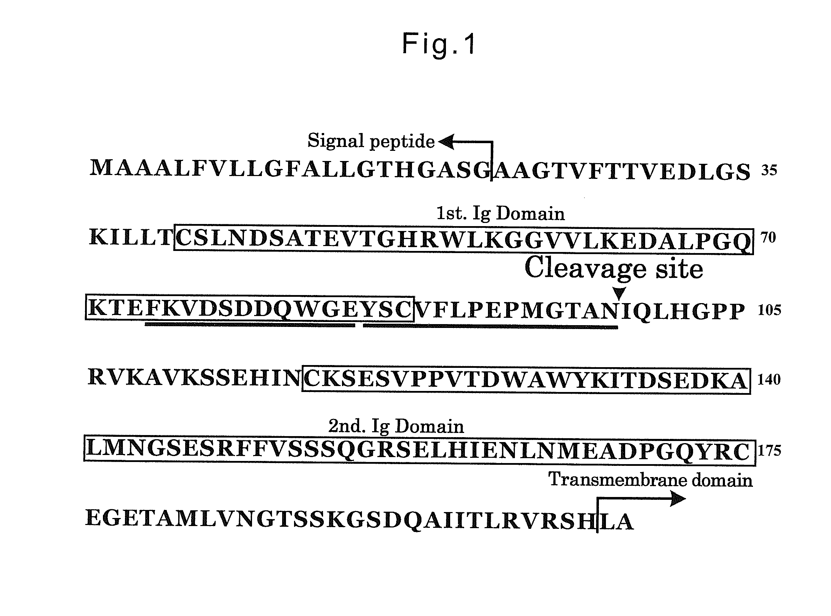

EMMPRIN is a single membrane-spanning glycoprotein with two Ig domains, and it has an expression-accelerating effect on collagenase (MMP-1). EMMPRIN null mice exhibit impaired spermatogenesis, fertilization, sensory function and memory function, as well as deficient mixed lymphocyte reaction. The primary structure of EMMPRIN is shown in Fig. 1, and the full-length sequence of EMMPRIN is listed as SEQ ID NO: 1. Ig domain 1 that is cleaved by MMP-1 induces expression of collagen.

The screening method of the invention comprises, but is not limited to, incubation of EMMPRIN and S100A9 in the presence of a candidate substance, and selection of a candidate drug that significantly inhibits binding between EMMPRIN and S100A9, as a chronic inflammation inhibitor or cancer metastasis inhibitor dependent on S100A9. As the evaluation criteria, for example, if binding between EMMPRIN and S100A9 proteins is inhibited at least 10%, or at least 20%, or at least 30%, or at least 50%, or at least 70% or 100% compared to a control, then chronic inflammation or cancer metastasis may be judged to be "significantly suppressed".

S100A9 often forms a complex with S100A8, as mentioned above, and the complex also binds with EMMPRIN. Thus, substances that inhibit binding between S100A8/A9 and EMMPRIN may be screened as chronic inflammation inhibitors or cancer metastasis inhibitors.

There are no particular restrictions on the means for detecting inhibition of binding between EMMPRIN and S100A9, and a calibration curve may be plotted for binding between EMMPRIN and S100A9 (or S100A8/A9) based on ELISA, with molecules that inhibit the binding, i.e. molecules that reduce absorbance, being detected as candidate drugs which are chronic inflammation inhibitors or cancer metastasis inhibitors. From the viewpoint of ensuring satisfactory detection sensitivity, the molecule adsorbed on the solid support is preferably EMMPRIN, which has a high molecular weight.

The term "chronic inflammation" as used herein encompasses atopic dermatitis and psoriasis, as well as cancer. Also, the term "inhibition of cancer metastasis " as used herein means inhibition of some or all of the processes involved including infiltration and migration from tissue in which cancer cell is the primary focus, through the blood and lymphatic vessels, establishment in new tissue, and initiation of proliferation, and it differs from growth inhibition of cancer cells.

### S100A8 and A9

The amino acid sequences of S100A8 and A9 and the DNA sequences coding for them have been published in Hum Genet (2002) 111:310-313 (NPL 10), for example. In general, the S100A8 and A9 to be used for the invention may be naturally occurring forms derived from humans, or recombinant proteins, so long as they are active, and they may be modified, heterogeneously derived or non-purified products. Recombinant proteins for S100A8 and A9 can be prepared in large amounts by methods known in the industry, for example, a method in which S100A8 or A9 gene (cDNA) that has been isolated or synthesized by PCR may be inserted into a plasmid or virus, for example, to prepare an expression vector, which is introduced into host cells, for example, cultured cells such as a microorganism, animal cells or plant cells, and expressed.

S100A9 is dissolved in water or culture medium, for example, culture medium suitable for culturing of epidermal keratinocytes, such as EpiLife™ culture medium, and added to the screening system of the invention. The addition amount cannot be specified for all cases, but it may be to a concentration of about 1 ng/ml to 1 mg/ml, preferably about 10 ng/ml to 100 µg/ml, and more preferably about 100 ng/ml to 10 µg/ml. The addition of S100A9 or S100A8/A9 is preferably carried out in the presence of calcium chloride. There are no particular restrictions on the culturing conditions such as the incubation time and incubation temperature in the presence of S100A9 or S100A8/A9, but preferably incubation is carried out with 5% CO₂, preferably at 30°C to 37°C for 1 to 14 hours and more preferably at 34°C to 37°C for 2 to 7 hours.

The chronic inflammation inhibitor of the invention can be utilized as a pharmaceutical or cosmetic effective for improvement, such as prevention or treatment, of persistent dermatitic conditions caused by S100A8/A9, such as atopic dermatitis or psoriasis.

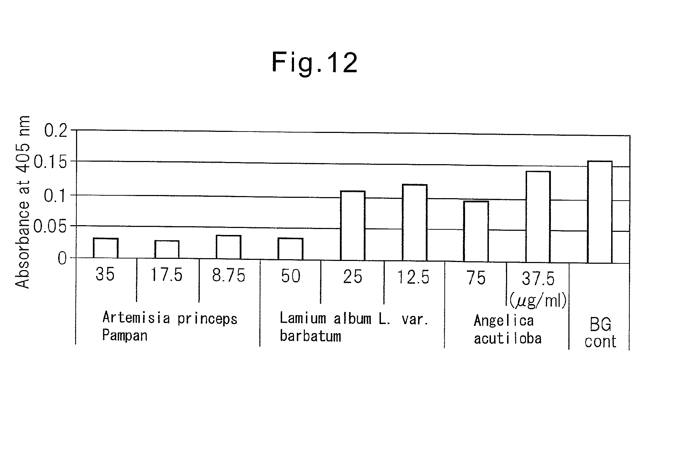

A chronic inflammation inhibitor or cancer metastasis inhibitor obtained by the screening method of the invention may be a plant body selected from the group consisting of Japanese mugwort, dong quai and white dead-nettle, or its extract. In particular, Japanese mugwort extract has been confirmed to significantly inhibit binding between S100A9 and EMMPRIN (Fig. 12), and is expected to be a suitable active ingredient for a chronic inflammation inhibitor or cancer metastasis inhibitor. Here, the plant body or extract from a plant to be used for the invention is any part of the plant body (flower, flowering spike, peel, fruit, stem, leaf, stalk, side leaf, trunk, bark, rhizome, root bark, root, seed or the entire plant) either directly or dried and pulverized into a dry powder, or extracted with a solvent from the plant part either directly or after drying and pulverizing.

For an extract, the extraction solvent used for extraction may be any solvent commonly used for extraction, and particularly there may be used alcohols such as methanol, ethanol or 1,3-butylene glycol, or organic solvents such as aqueous alcohols, acetone or ethyl acetate, either alone or in combinations, among which alcohols and aqueous alcohols are especially preferred, with methanol, ethanol, 1,3-butylene glycol, aqueous ethanol and aqueous 1,3-butylene glycol being most preferred. The solvent is preferably used at room temperature or a temperature below the boiling point of the solvent.

The extraction process is not particularly restricted, but it will usually be carried out in a range from ordinary temperature to the boiling point of the solvent under ordinary pressure, and following extraction, filtration or an ion exchange resin may be used for adsorption, decoloration and purification into a solution, paste, gel or powder form. In most cases this form may be utilized directly, but if necessary, purifying treatment such as deodorization or decoloration may be performed, within ranges that do not alter the effect, and the means for purifying treatment such as deodorization or decoloration may be an active carbon column or the like, with commonly employed means being selected as appropriate and desired depending on the extracted substance.

The extraction site of the plant body may be leaves in the case of Japanese mugwort, roots in the case of dong quai, or stems, leaves or flowers in the case of white dead-nettle, but there is no restriction on the extraction site.

The extract obtained by extraction with the solvent may be used directly, or for example, after concentration by freeze-drying Alternatively, if necessary, the impurities may be removed by an adsorption method using an ion exchange resin, for example, or it may be subjected to adsorption on a porous polymer (for example, AMBERLITE XAD-2) column and then eluted with a desired solvent and further concentrated.

The chronic inflammation inhibitor or cancer metastasis inhibitor of the invention is preferably one comprising one or more plant bodies or their extracts, but other components may also be included so long as the effect of the invention is not impaired. The dosage, method of administration and dosage form of a chronic inflammation inhibitor or cancer metastasis inhibitor of the invention may be appropriately established according to the purpose of use. For example, the dosage form of a chronic inflammation inhibitor of the invention may be oral, parenteral or external. As examples of dosage forms there may be mentioned oral administration forms such as tablets, powders, capsules, granules, extract agents and syrups, parenteral administration forms such as injections, drops and suppositories, and external preparations such as ointments, creams, emulsions, lotions, packs and bath additives.

The content of the extract component in the chronic inflammation inhibitor or cancer metastasis inhibitor of the invention may be appropriately established according to the purpose of use, but generally it will be 0.0001 to 20.0 mass% and preferably 0.0001 to 10.0 mass% as dry product in the total inhibitor. Japanese mugwort extract, white dead-nettle extract and dong quai extract are believed to suppress chronic inflammation or cancer metastasis in a concentration-dependent manner.

The chronic inflammation inhibitor or cancer metastasis inhibitor may contain, in addition to the aforementioned drugs, excipients, moisture-proof agents, antiseptic agents, toughening agents, thickeners, emulsifiers, antioxidants, sweeteners, acidulants, seasonings, coloring agents, flavorings and the like, which are commonly used in foods and pharmaceuticals, skin whiteners, humectants, oil components, ultraviolet absorbers, surfactants, thickeners, alcohols, powder constituents, pigments and aqueous components, water and various skin nutrient preparations, which are commonly used in cosmetics and the like, in appropriate amounts as necessary.

When the chronic inflammation inhibitor or cancer metastasis inhibitor of the invention is to be used as an external preparation for skin, there may be appropriately added auxiliary agents that are commonly employed in external preparation for skins, for example, metal sequestering agents such as disodium edetate, trisodium edetate, sodium citrate, sodium polyphosphate, sodium metaphosphate and gluconic acid, drugs such as caffeine, tannin, verapamil, tranexamic acid and its derivatives, licorice extract, glabridin, hot water extract of Chinese quince fruit, various galenicals, tocopherol acetate and glycyrrhizinic acid and its derivatives or salts, skin whiteners such as vitamin C, magnesium ascorbate phosphate, ascorbic acid glucoside, arbutin and kojic acid, saccharides such as glucose, fructose, mannose, sucrose and trehalose, and vitamin A compounds such as retinoic acid, retinol, retinol acetate and retinol palmitate.

Concrete examples will now be provided for a more detailed explanation of the invention. However, the invention is in no way limited by the examples.

### [Examples]

### 1. Screening for novel S100A9 receptor candidates

After mixing a protein mixture recovered from cultured keratinocytes with GST-fused S100A9 or S100A8/A9 protein, the sample was used for comprehensive analysis of the proteins by capillary LC/MS/MS.

### LC/MS/MS analysis

Silica frit was prepared to hold the filler, at the edge of the tapered outlet end of a non-packed capillary column (product of New Objective) with an inner diameter of 100 µm and a length of 120 mm. In the obtained capillary column there was packed the octadecylated silica-type filler Aqua C18 (product of Phenomenex) with a mean particle size of 5 µm, to a height of 100 mm, to obtain a reverse-phased capillary column for analysis.

Silica frit was prepared to hold the filler, at the edge of the outlet end of a non-packed capillary column (product of Agilent) with an inner diameter of 250 µm and a length of 150 mm. Through the outlet end of the obtained capillary column there was packed a mixture of the cation-exchange resin-type filler Partisphere SCX resins (product of Whatman) having a mean particle size of 5 µm, and the anion-exchange resin-type filler PolyWAX LP (product of PolyLC) having a mean particle size of 5 µm, at a weight ratio of 2:1, and at the inlet end there was packed the octadecylated silica-type filler Aqua C18 (product of Phenomenex) having a mean particle size of 5 µm, each to a height of 25 mm, to obtain a biphasic capillary column comprising trap-type reversed-phased capillary column and an SCX-WAX-mixed capillary column.

During preparation of the reversed-phase capillary column for analysis and the biphasic capillary column, high pressure nitrogen gas and a pressurized packing vessel were used for packing of the filler by a slurry packing method.

The protein mixture was then analyzed by 6-step MudPIT-type analysis (two-dimensional HPLC/ESI MS/MS).

First, a supernatant containing approximately 4 µg of peptide was loaded into a biphasic capillary column by a pressure method, and then mobile phase A (a liquid mixture of water, acetonitrile and formic acid in a volume ratio of 95:5:0.1, pH approximately 2.6) at a volume of at least 10 times the sample solution was used for rinsing and desalting. The biphasic capillary column 10 was connected to a reversed-phase capillary column for analysis 20 through a perforated union (product of Upchurch Scientific) (not shown). Next, it was connected to a Nanospace SI-2 HPLC apparatus (Shiseido Corp.) using a capillary with an inner diameter of 100 µm was used as tubing. Here, the trap-type reversed-phase capillary column 11 was situated at the upstream end of the SCX-WAX mixing capillary column 12 and the reversed-phase capillary column for analysis 20.

The mobile phases used were mobile phase A, mobile phase B (a mixture of water, acetonitrile and formic acid in a volume ratio of 20:80:0.1) and mobile phase C (mobile phase A containing 500 mM ammonium acetate; pH: approximately 6.8), and the peptide elution method was a gradient elution method with a total of 6 steps, with the vol% of the mobile phase C, in a rectangular fashion, being incrementally increased at each step.

The gradient profile for step 1 was: flow of mobile phase A for 5 minutes followed by increase in the proportion of mobile phase B from 0 vol% to 15 vol% for 5 minutes, increase in the proportion of mobile phase B to 45 vol% for 60 minutes, increase in the proportion of mobile phase B to 75 vol% for 10 minutes, and then flow for 5 minutes at that proportion.

The gradient profile for steps 2 to 6 was: flow of mobile phase A for 1 minute, followed by flow with the proportion of mobile phase C at X [vol%] for 4 minutes, increase in the proportion of mobile phase C from 0 vol% to 15 vol% for 5 minutes, increase in the proportion of mobile phase C to 45 vol% for 60 minutes, increase in the proportion of mobile phase C to 75 vol% for 10 minutes, and then flow for 5 minutes at that proportion. During this time, the flow rate of liquid conveyance by the pump was 250 µL/min, and the flow rate of the column was adjusted to 300-400 nL/min by splitting with a resistance capillary.

For measurement of ESI MS/MS, an ion trap mass spectrometer LCQ-Deca (product of Thermo Fisher Scientific) was used. During this time, the peptide eluted from the reversed-phase capillary column for analysis was directly introduced into the mass spectrometer without splitting.

There were repeated, through each of the steps, full scan MS spectroscopic measurement once, with a mass-to-charge ratio (m/z) of 400-1400, and data-dependent MS/MS spectroscopic measurement three times. The standardized bond dissociation energy was 35%. Microscan 3 was used for both the MS spectroscopic measurement and MS/MS measurement. Also, the dynamic exclusion settings were: a repeat count of 1, a repeat period of 0.50 minute, an exclusion list size of 25 and an exclusion period of 10.00 minutes.

A search was conducted for the obtained MS/MS spectrum in a non-redundant human database (ftp://ftp.ncbi.nih.gov/blast/db/FASTA/nr.gz, Ver.2007/2/8), using the SEQUEST algorithm running on Bioworks software (product of Thermo Fisher Scientific).

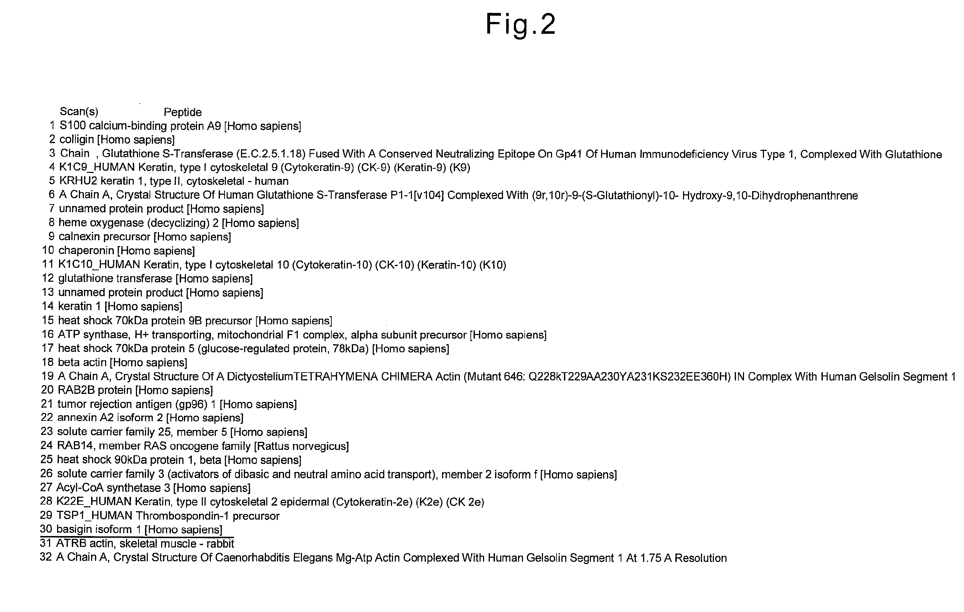

As a result, receptor candidate proteins were identified, as listed in Fig. 2. Of these proteins, Basigin (EMMPRIN) was tested as follows, as a novel receptor for S100A9.

### 2. Expression-suppressing effect on EMMPRIN by siRNA

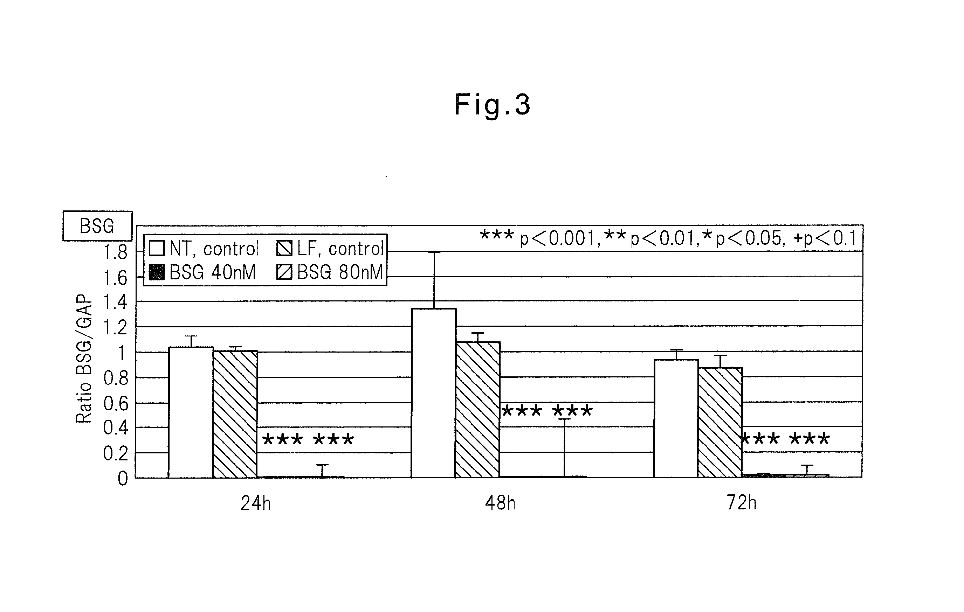

In order to suppress expression of EMMPRIN, EMMPRIN siRNA (Santa Cruz: sc-35298) was transfected into cultured keratinocytes in the growth-phase to a final concentration of 40 nM or 80 nM, using RNAiMax ("BSG" in Fig. 3). As controls there were used a sample without addition ("NT" (non-treated control) in Fig. 3), and control siRNA-A (Santa Cruz Biotechnology, Inc., sc-3707) having no homology with any portion of the human gene ("LF" in Fig. 3). The transfection was carried out by exchanging the culture medium with basal medium containing no growth factor. At 24 hours after transfection, the growing keratinocytes were stimulated with S100A9, and after an elapse of 24 hours, the RNA was recovered. As a result, as shown in Fig. 3, transfection of EMMPRIN siRNA suppressed expression by 1-3% after 24, 48 and 72 hours, compared to the expression level of EMMPRIN using the control.

### 3. Changes in cytokine and MMP expression produced by suppressed EMMPRIN expression

It has been demonstrated that IL-8 (CXCL-8), TNFα, IL1-F9 and CXCL-1 are expressed in an accelerated manner in keratinocytes by addition of S100A8/A9 (Ibid, J Cell Biochem. (2008) 104:453-464) (NPL 8). Using real-time quantitative PCR, it was then investigated whether addition of S100A9 also accelerates expression of IL-8 (CXCL-8), TNFα, IL1-F9 and CXCL-1, and whether suppressing expression of EMMPRIN has any effect on expression of these cytokines. The same method was also used to examine the effect of S100A9 stimulation on MMP-1 and MMP-10 expression.

### Real-time quantitative PCR

Growth-phase NHEK, cultured in EpiLife™-KG2 (Cascade Biologies) were exposed for 3 hours after exchanging to the same culture medium containing and not containing 2 mM calcium chloride and S100A8 or S100A9 (10 µg/ml each), and a MagNA™ Pure mRNA extraction kit and a MagNA Pure™ device (Roche Diagnostics, Tokyo, Japan) were used for extraction of the mRNA. The obtained mRNA was reverse transcripted using SuperScript™ II (Invitrogen Corporation, Carlsbad, California, U.S.). Real-time quantitative PCR was performed on a LightCycler highspeed thermal cycler system using a LightCycler FastStart DNA master SYBR green I kit (Roche Diagnostics), according to the manufacturer's instructions. Typical reaction conditions are 40 cycles, comprising an activation step for 10 minutes followed by denaturation at 95°C for 15 seconds, annealing at 60°C for 10 seconds and extension at 72°C for 10 seconds. The primers used are shown in Table 1 below. The final concentration of each primer was 0.2-0.25 µM in a total reaction volume of 20 µl. The glyceraldehyde 3-phosphate dehydrogenase (GAPDH) gene was used as the control gene. The specificity of the amplified fragment was confirmed by melting curve analysis. The expression level of each gene was quantitatively analyzed using LightCycler analysis software (NPL 11: Morrison TB et al., Biotechniques (1998)24:954-958, 960, 962). The amount of the target mRNA was shown as a ratio with respect to the amount of mRNA of an A8/control siRNA-A (Santa Cruz: sc-37007) (A8/LF).

**[Table 1]**

| Primers used in quantitative RT-PCR | | | |
|---|---|---|---|
| Gene | Forward primer | Reverse primer | Length |
| IL-8 | TCAGAGACAGCAGAGCACACA (SEQ ID NO 2) | AATCAGGAAGGCTGCCAA(SEQ ID NO 3) | 127 bp |
| TNFα | GACAAGCCTGTAGCCCATGT (SEQ ID NO 4) | TTGATGGCAGAGAGGAGGTT(SEQ ID NO 5) | 266 bp |
| IL1F9 | AGGAAGGGCCGTCTATCAAT (SEQ ID NO 6) | AATGTGGGCTGTTCTCOCAAC(SEQ ID NO 7) | 258 bp |
| CXCL1 | AACCGAAGTCATAGCCACAC (SEQ ID NO 8) | GTTGGATTTGTCACTGTTCAGC (SEQ ID NO 9) | 109 bp |
| S100A8 | GGGCAAGTCCGTGGGCATCATGTTG (SEQ ID NO 10) | CCAGTAACTCAGCTACTCTTTGGGCTTTCT (SEQ ID NO 11) | 313 bp |
| S100A9 | GCTCCTCGGCTTTggGACAGAGTGCAAG (SEQ ID NO 12) | GCATTTGTGTCCAGGTCCTCCATGATGTGT (SEQ ID NO 13) | 240 bp |
| GAPDH^{h} | GAGTCAACGGATTTGGTCGT (SEQ ID NO 14) | TGGGATTTCCATTGATGACA (SEQ ID NO 15) | 200 bp |

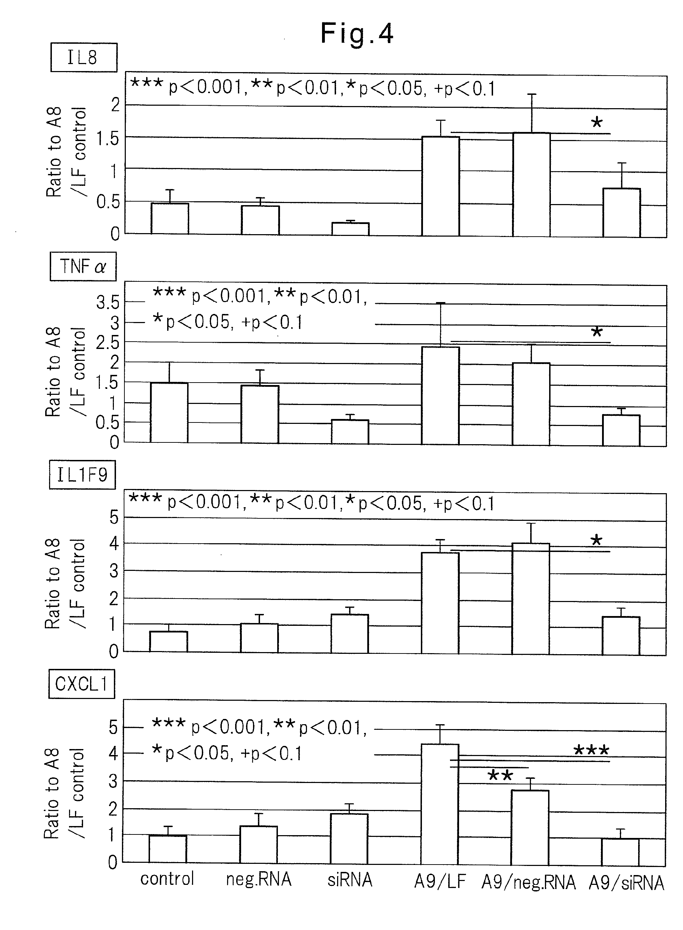

As shown in Fig. 4, the S100A9-added sample (A9/LF) induced expressed of all of the cytokines. On the other hand, the EMMPRIN siRNA-added sample (A9/siRNA) significantly reduced the expression levels of all of the cytokines. This clearly suggests that when EMMPRIN expression is suppressed by EMMPRIN siRNA, cytokine expression is suppressed even when cytokine expression is stimulated with S100A9.

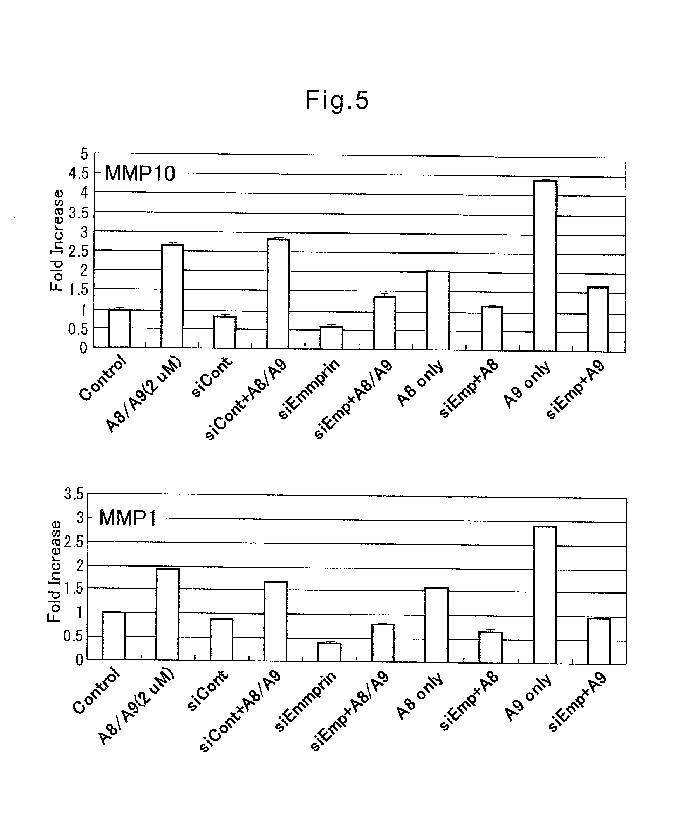

It was also demonstrated that addition of S100A9 accelerated expression of MMP as well, whereas knockdown of EMMPRIN significantly suppressed expression even with addition of S100A9 (Fig. 5).

### 4. EMMPRIN and S100 protein binding test

### 1) Construction of EMMPRIN extracellular domain

### [Method]

Cells: A human fetal kidney cell line (HEK293) purchased from ATCC, and cultured human normal fibroblasts OUMS-24 isolated by Prof. Masayoshi Nanba. The HEK293 and OUMS-24 were cultured using DMEM/F12 culture medium by Gibco (with addition of fetal calf serum to a final concentration of 10%).

### 2) EMMPRIN extracellular domain expression construct:

A PDNR 1r vector (promoterless donor vector, product of Clontech) having CMV intron promoter (CMVi) introduced therein was constructed, and cDNA coding for the human EMMPRIN extracellular domain (with a myc-HA-Flag-6His tag added at the C-terminus) was inserted downstream from CMVi (pCMVi-exEmmp: EMMPRIN extracellular domain-expressing donor vector). The nucleotide sequence of the inserted cDNA has been confirmed to be correct by a DNA sequencer.

### 3) Extracellular secretion of EMMPRIN extracellular domain:

After inserting pCMVi-exEmmp into HEK293 using the transfection reagent FuGENE-HD (Roche) and cultured for 48 hours, the culture supernatant was collected. An anti-HA tag antibody covalently bonded carrier by Sigma was added to the culture supernatant, and the mixture was shake-mixed at 4°C for 3 hours. It was then centrifuged at 5000 rpm for 1 minute, and the precipitating carrier-bonded protein was eluted with acidic buffer. The eluted sample was electrophoresed using 12% SDS-PAGE, and then electroblotted on a PVDF membrane and anti-HA tag antibody by CST Co. was used for Western blotting, thus confirming secretion of the EMMPRIN extracellular domain.

### 4) EMMPRIN extracellular domain-expressing adenovirus (Ad-exEmmp):

Conversion of pCMVi-exEmmp to an adenovirus vector was accomplished using an adenovirus construction kit (Adeno-X-expression system: Clontech).

### 5) Large-volume purification of EMMPRIN extracellular domain

Cultured human normal fibroblasts OUMS-24 (10 cm dish x 20) were infected with Ad-exEmmp (20 MOI). The infection period was during high density OUMS-24. This is because no cell division occurs with cells that have been brought to contact inhibition by high density culturing, and decline in the episome content of adenovirus present in the cells is suppressed, such that target gene expression by the adenovirus is continuous for a very long period (2 to 3 weeks). Furthermore, since OUMS-24 is capable of serum-free culturing, the recombinant protein secreted in the culture supernatant over long periods can be recovered in a condition without serum. After the infection procedure, culturing is carried out for 24 hours and the serum-free medium is exchanged with DMEM/F12 (phenol red-free). With night exchange at 3-day intervals, the culture supernatant was collected each time and stored at 4°C (varying the storage conditions depending on the stability of the protein). The procedure was carried out for 30 days. An approximately 2 L portion of recovered culture supernatant, obtained as a precipitate under 80% saturated ammonium sulfate conditions, was dissolved in 50 ml of purified water, and then the ammonium sulfate was removed by dialysis against purified water. Following dialysis, the recombinant protein of interest was recovered using an anti-HA tag antibody covalently bonded carrier-packed column (Sigma).

### 6) Identification of EMMPRIN-binding proteins:

EMMPRIN-binding proteins were identified by immunoprecipitation and Western blotting, for confirmation that EMMPRIN is a novel receptor for S100 protein. The EMMPRIN used for the experiment had a myc-HA-Flag-6His tag attached to the C-terminus. The S100 proteins used were S100A8 and S100A9 protein. HEK293 cells were transfected with plasmids coding for the proteins (with HA tags attached to the C-termini), and the proteins were isolated from the respective culture supernatants.

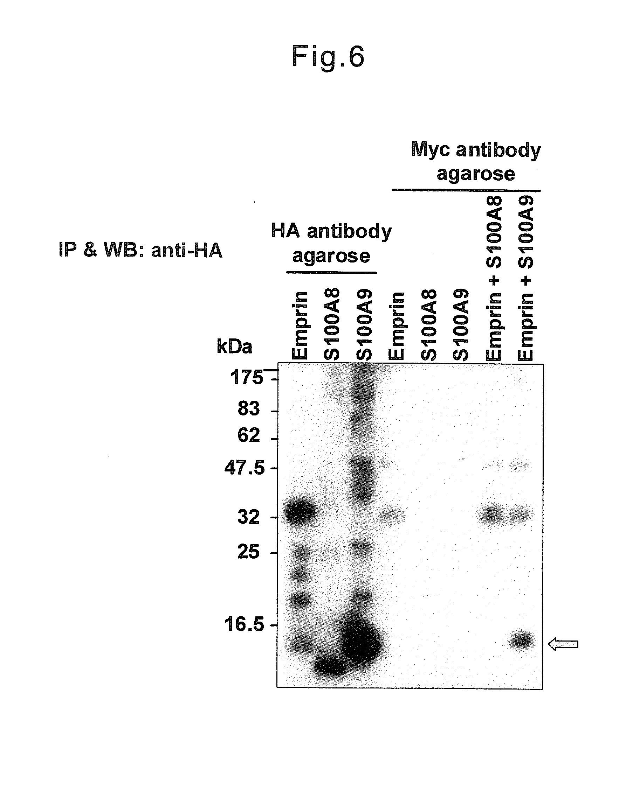

In order to analyze binding between EMMPRIN and S100A8 and S100A9 protein, the culture supernatants containing the respective proteins were mixed and reacted, and then HA antibody and Myc antibody were used for immunoprecipitation. The results of Western blotting are shown in Fig. 6. For the EMMPRIN and S100A8 mixture sample, only a band for EMMPRIN near 32 kDa was confirmed ("EMMPRIN + S100A8"). For the EMMPRIN and S100A9 protein mixture sample ("EMMPRIN + S100A9"), a band representing their bound form was confirmed near 47.5 kDa. These results demonstrated that EMMPRIN is a novel receptor candidate for S100A9 protein.

### 5. Effect of soluble EMMPRIN on MMP expression

Accelerated expression of MMP has been thought to be due to autocrine secretion of EMMPRIN, in which the extracellular domain of EMMPRIN is decomposed by MMP, and released soluble EMMPRIN binds to EMMPRIN present as a receptor on cell surfaces, promoting production of MMP. It was therefore investigated whether soluble EMMPRIN actually accelerates expression of MMP.

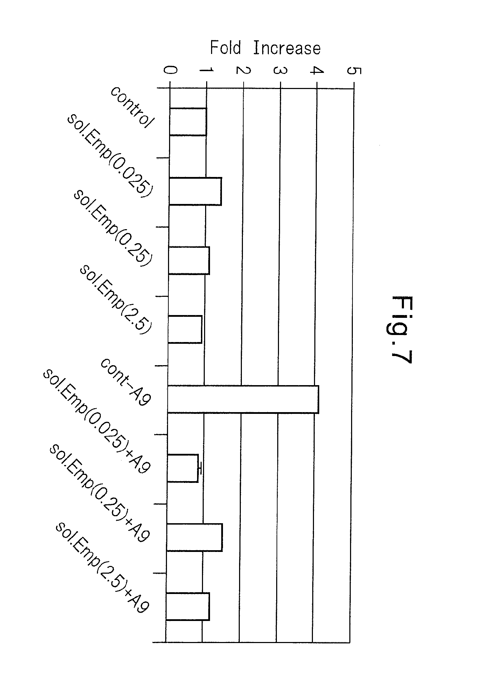

The soluble EMMPRIN used was EMMPRIN extracellular domain purified by the method described above, which when added to keratinocytes exhibited virtually no MMP-1-inducing effect at concentrations of 0.025, 0.25 and 2.5 µM. Also, while MMP-1 expression was notably accelerated with S100A9 alone, addition of both soluble EMMPRIN and S100A9 to keratinocytes significantly suppressed the MMP1 expression-accelerating effect of S100A9. The results are shown in Fig. 7. It is believed that MMP expression was suppressed when soluble EMMPRIN and S100A9 were both present because S100A9 which induces MMP production was scavenged by soluble EMMPRIN, with both forming a bonded complex. Based on these results, it is concluded that a mechanism whereby S100A9 stimulation accelerates MMP expression via EMMPRIN, is more reasonable than the conventionally proposed autocrine mechanism of EMMPRIN. Although the results are not shown here, soluble EMMPRIN also significantly suppressed MMP-10, TNFα and IL-8 expression.

### 6. Localization of EMMPRIN in epidermis

### 1) Immunostaining

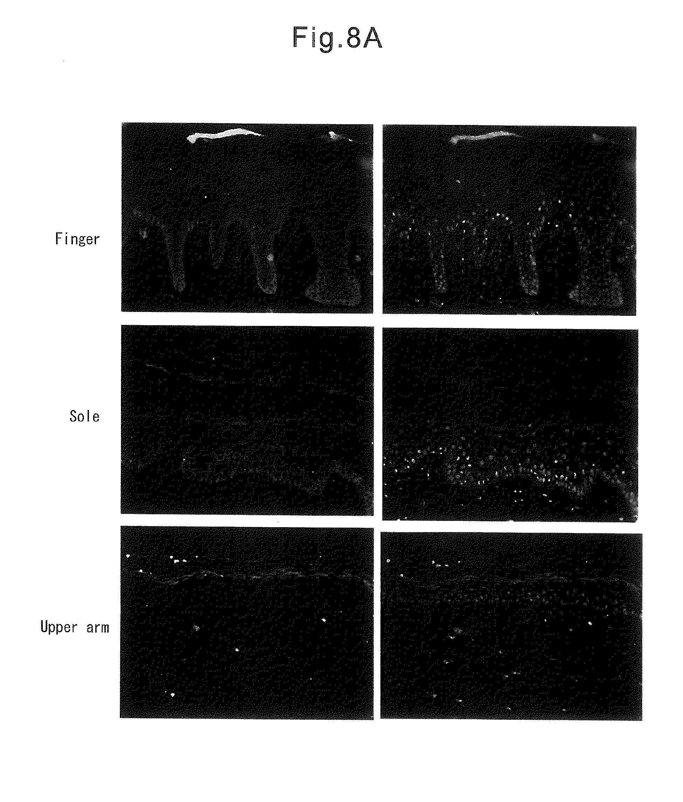

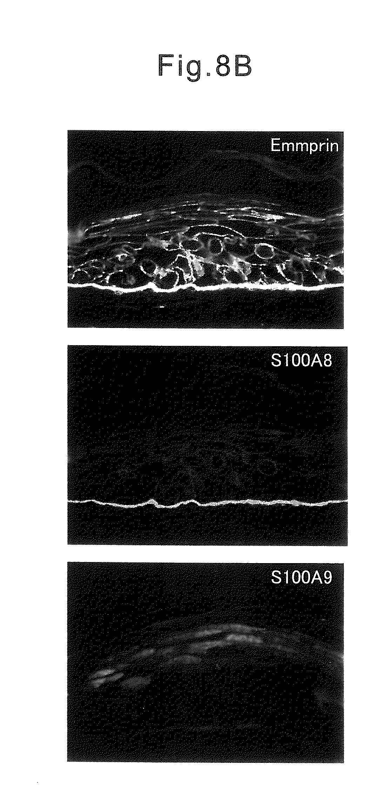

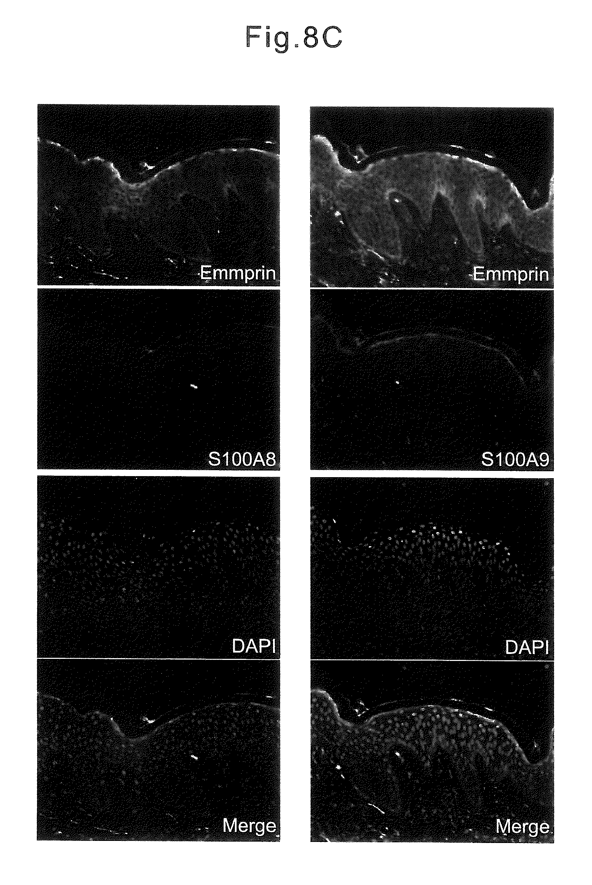

By immunostaining it was confirmed whether EMMPRIN is present in human epidermis or in the same localized region as S100 protein. The results are shown in Fig. 8A to C. EMMPRIN is abundantly expressed in the granular layer of skin from normal epidermis, skin models and atopic dermatitis (AD). S100 protein is also expressed in the vicinity of EMMPRIN.

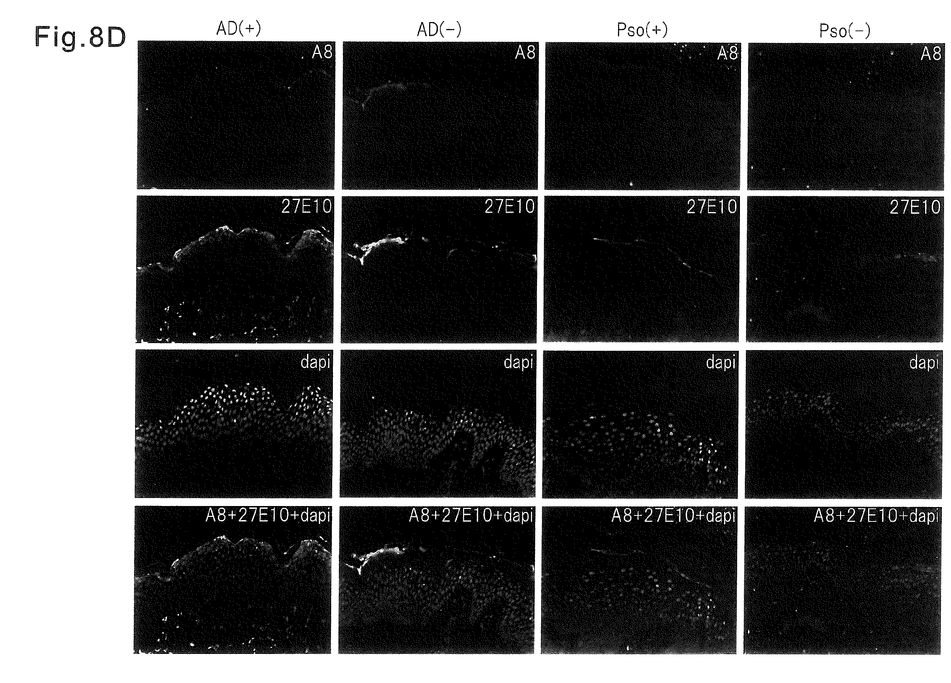

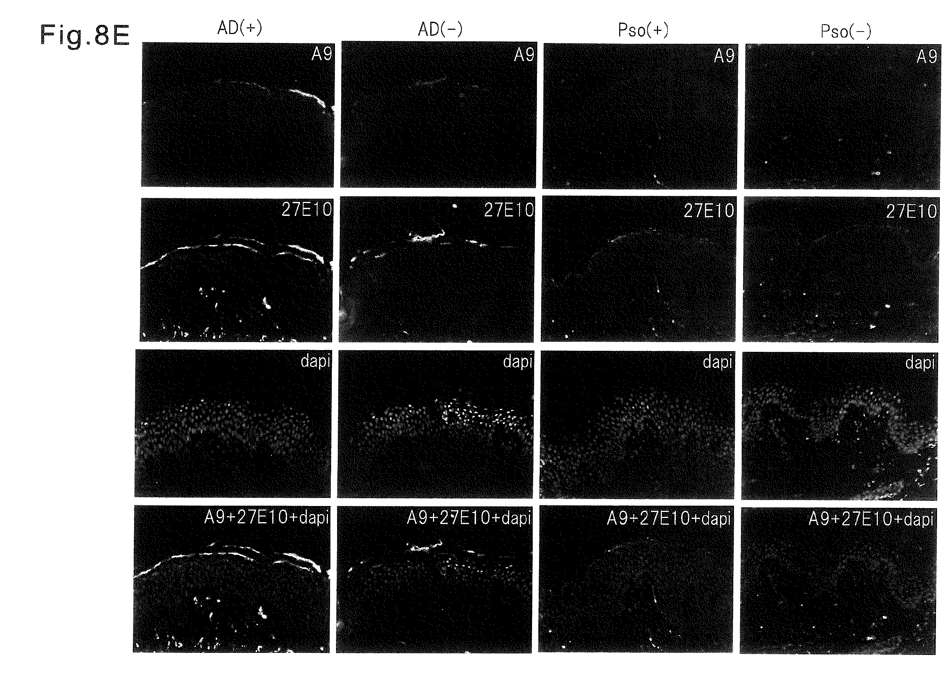

It has also been demonstrated that EMMPRIN is highly expressed, and S100A8 and S100A9 protein expression is accelerated, in atopic dermatitis skin. The results of immunostaining using 27E10 antibody that specifically binds to S100A8/A9 complex, indicated that compared to psoriasis (Pso) skin, S100A8/A9 complex is more highly expressed in the granular layer of atopic diseased skin (Fig. 8D and Fig. 8E).

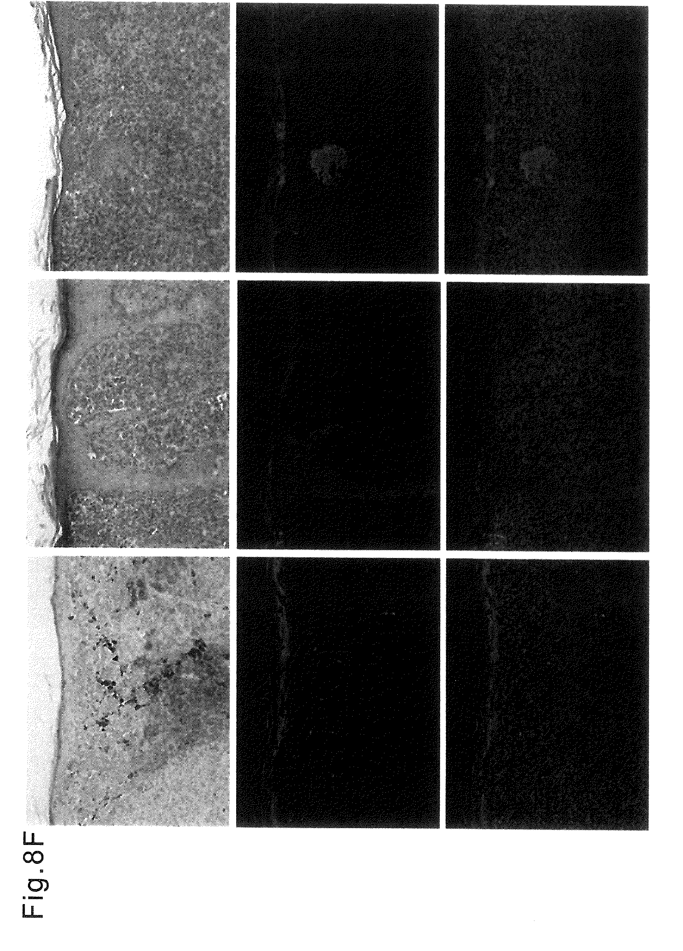

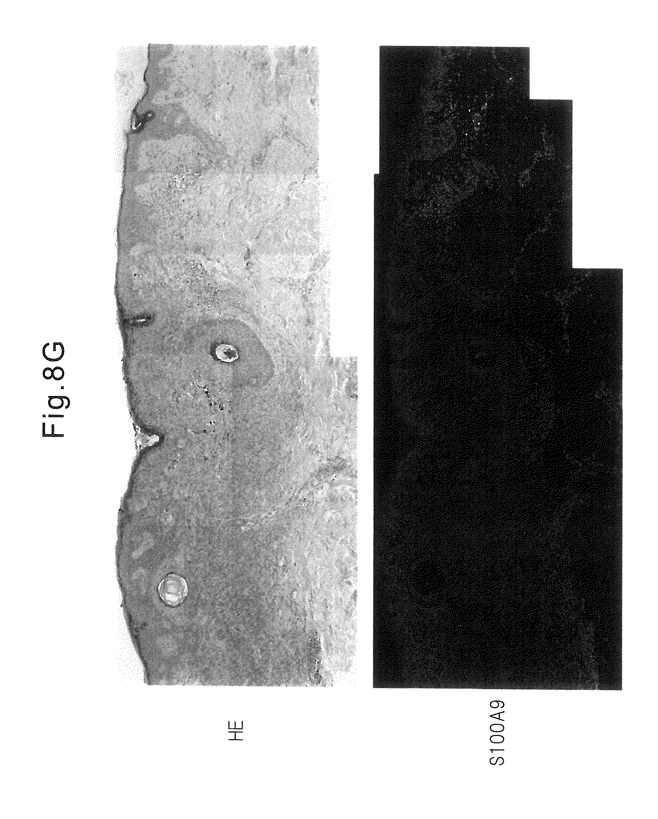

The results of immunostaining showed virtually no expression of S100A8, A9 or EMMPRIN in normal epidermis. As confirmed by immunostaining for S100A9 expression in melanoma tissue, however, S100A9 overexpression was found in the epidermis of all the samples (Fig. 8F, left, center and right photographs). Also, while no S100A9 was expressed at normal sites, with malignant melanoma (Clark's level III), it was confirmed that S100A9 is expressed in the epidermis directly above the basal lamina corresponding to infiltration of melanoma cells (Fig. 8G). On the other hand, no S100A9 expression was found in the epidermis in mole tissue that was present in the same tumor mass.

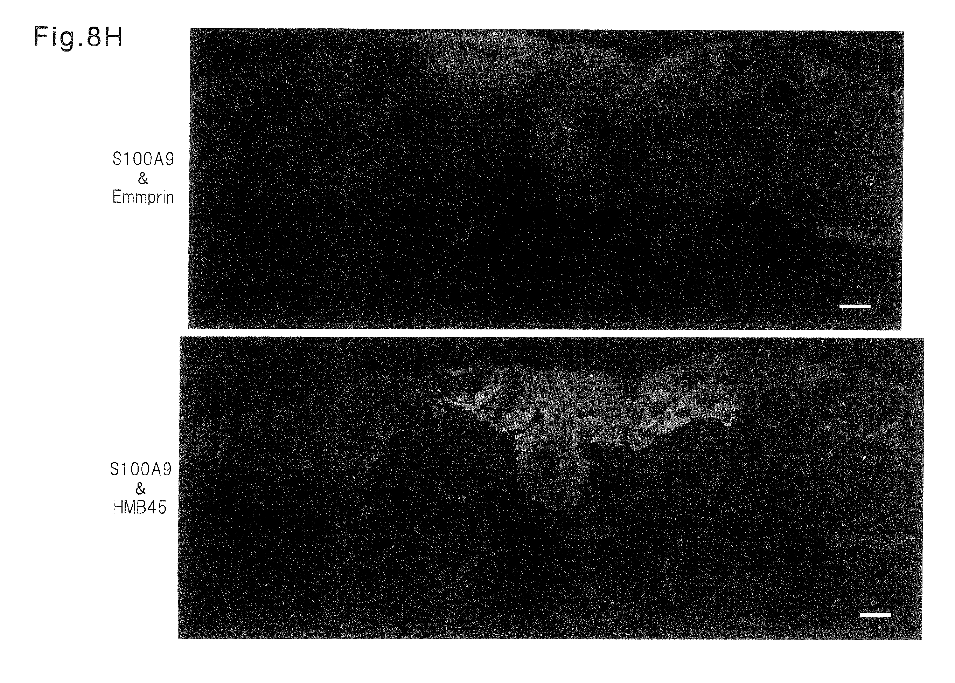

At the sites that were immunostained using S100A9 antibody and EMMPRIN (CD147) antibody, when stained with melanoma specific antibody (HMB45) instead of EMMPRIN antibody, the sites that stained by melanoma specific antibody overlapped with EMMPRIN antibody (Fig. 8H). This result confirmed that EMMPRIN is expressed in infiltrating melanoma cells.

### 2) Confirmation of interaction between EMMPRIN and S100A9 in atopic skin

It was confirmed by PLA (Proximity Ligation Assay) that EMMPRIN and S100A9 protein do not simply bind, but actually interact. With the PLA method it is possible, by hybridizing complementary DNA labeled with a fluorescent dye, and using two types of DNA probe-labeled antibodies, to demonstrate whether or not these proteins interact. The PLA method has much higher sensitivity than common immunostaining.

A Duolink in *situ* PLA kit by Olink Co. was used for the interaction test. Skin tissue of an affected area obtained from an atopic patient was fixed with 4% paraformaldehyde and then embedded in paraffin by a common method. After slicing to 4 µm, it was subjected to xylene treatment and ethanol treatment and rinsed with PBS, and then blocked and reacted overnight with a primary antibody (see Table 1 below) at 4°C. After rinsing with PBS, it was reacted with PLA probes (see Table 1) at 37°C for 2 hours.

**[Table 2]**

| | Tissue | Primary antibody | PLA probe |
|---|---|---|---|
| (1) | Atopic skin disease lesion | Goat anti-S100A9 (Santa Cruz) + mouse anti-EMMPRIN (HIM6) (BioLegend) | Goat PLUS + mouse MINUS |
| (2) | Atopic skin disease lesion | Goat anti-S100A9 (Santa Cruz) + mouse anti-EMMPRIN (HIM6) (BioLegend) | Goat PLUS + mouse MINUS |
| (3) | Atopic skin disease lesion | Mouse anti-calprotectin (Hycult biotechnology: 27E10) + goat anti-EMMPRIN (K-20) (Santa Cruz) | Goat PLUS + mouse MINUS |

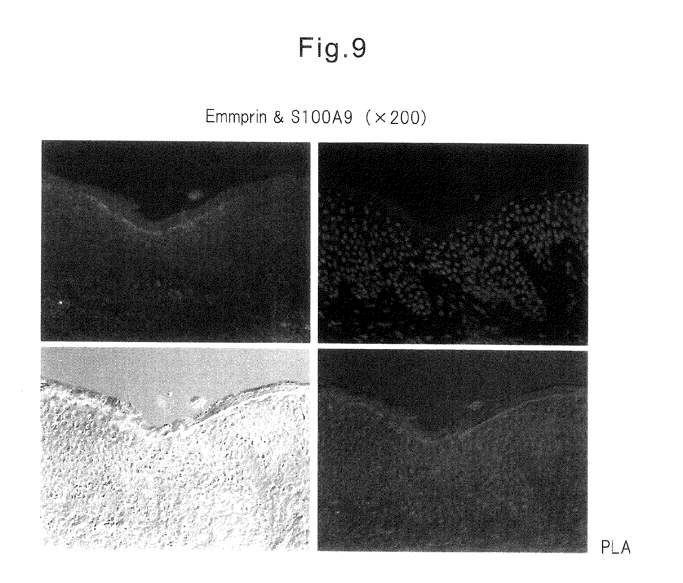

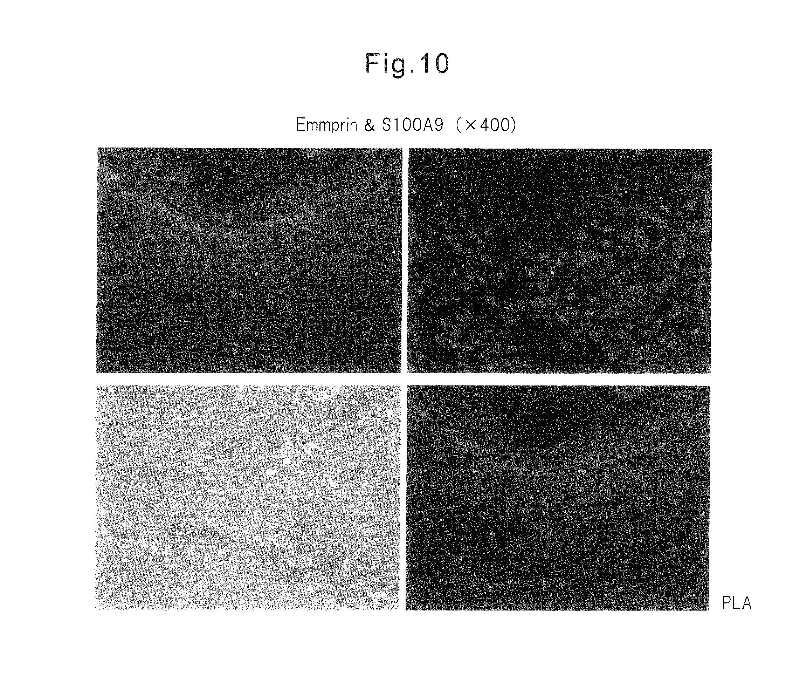

After rinsing, it was hybridized with DNA probes, rinsed with TBS-T, and then ligase was added, and the mixture was incubated at 37°C for 15 minutes for fusion of the probes. Polymerase was added, the mixture was incubated at 37°C for 90 minutes, and the ligated DNA probe was amplified. Detection kit 613 (Olink) was used for fluorescent dye labeling, followed by microscopic observation. The results are shown in Fig. 9 and Fig. 10.

When PLA was carried out with EMMPRIN and S100A9 antibody, a strong positive reaction was observed from the stratum spinosum to near the granular layer (Fig. 9). This indicates that EMMPRIN and S100A9 interact.
Positive reaction was also observed near the granular layer for S100A8, but this was presumably a result of formation of dimers with S100A9 (results not shown).

### 7. Screening for drugs that inhibit binding of S100A8 and S100A9 proteins to the EMMPRIN extracellular domain.

### 1) Preparation and biotinylation of recombinant S100A8, S100A9

Human S100A8 and S100A9 were produced in E. *coli* as GST fused proteins, and purified by affinity chromatography using a glutathione covalently bonded carrier. The GST was the cut off and removed. The purified S100A8 and S100A9 proteins were biotinylated by the following method. A 3-fold molar amount of Biotin-(AC5)2Sulfo-OSu (Dojindo) was mixed with each purified protein concentration. After reaction at room temperature for 2 hours, Nap-5 (GE Healthcare) was used to remove the unreacted biotinylating reagent.

### 2) Screening for drugs that inhibit binding of S100A8 and S100A9 proteins to the EMMPRIN extracellular domain:

Recombinant EMMPRIN extracellular domain (corresponding to the portion after the signal peptide and before the transmembrane domain in Fig. 1) was bound to the wells of a 96-well plate (Pierce). After rinsing the wells, each well was treated with 5% BSA and another blocking agent to minimize nonspecific adsorption. The test drug (or the solvent alone as a control) was then added to each well and incubated at room temperature for one hour. After rinsing the wells, recombinant S100A9w (the full-length sequence of EMMPRIN) was added and incubated at room temperature for 1 hour. Also, HRP-labeled anti-S100A9 antibody was added to and reacted in the same well. After another rinsing, chromogenic substrate (ortho-phenylenediamine) was added, and the absorbance (O.D. 492 nm) was measured with an ELISA reader. In the procedure, first a calibration curve for binding between EMMPRIN and S100A9 (or S100A8/A9) was plotted. Molecules that inhibit the binding were then screened. Drugs were added to the assay system, and those that lowered absorbance were noted as candidate drugs.

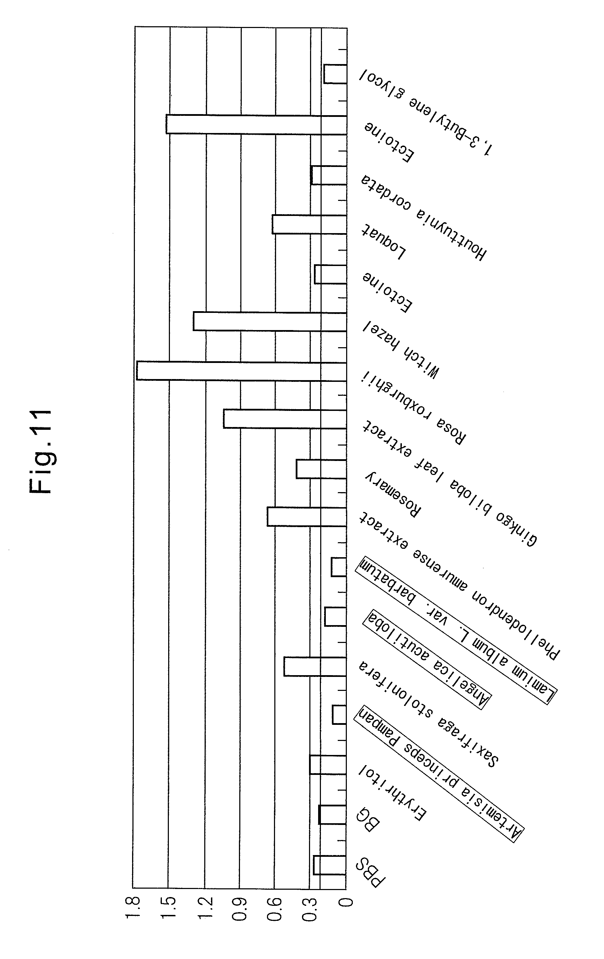

When different plant extracts were subjected to this screening method, Japanese mugwort extract, dong quai extract and white dead-nettle extract were found to inhibit binding between EMMPRIN and S100A9 to a significant degree compared to the control (Fig. 11). Among them, Japanese mugwort extract exhibited the strongest inhibiting effect (Fig. 12).

## Claims

1. A screening method for chronic inflammation inhibitors or cancer metastasis inhibitors wherein, when a candidate substance as a chronic inflammation inhibitor or cancer metastasis inhibitor significantly inhibits binding between EMMPRIN and S100A9 or S100A8/A9, the candidate substance is evaluated as significantly suppressing chronic inflammation or cancer metastasis.

2. The screening method for chronic inflammation inhibitors or cancer metastasis inhibitors according to claim 1, which comprises incubating EMMPRIN and S100A9 or S100A8/A9 in the presence of a candidate substance as a chronic inflammation inhibitor or cancer metastasis inhibitor, and selecting a substance that inhibits binding between EMMPRIN and S100A9 or S100A8/A9 as a chronic inflammation inhibitor or cancer metastasis inhibitor.

3. The method according to claim 1 or 2, wherein the EMMPRIN is solid phased on a solid support.

4. The method according to any one of claims 1 to 3, wherein the inhibition of binding is determined by ELISA.

5. A chronic inflammation inhibitor or cancer metastasis inhibitor comprising a drug that inhibits binding between EMMPRIN and S100A9 or S100A8/A9.

6. The chronic inflammation inhibitor or cancer metastasis inhibitor according to claim 5, wherein the drug comprises one or more plant bodies selected from the group consisting of Japanese mugwort, dong quai and white dead-nettle, or their extracts.

7. A method of suppressing chronic inflammation or cancer metastasis, which comprises administering a drug that inhibits binding between EMMPRIN and S100A9 or S100A8/A9 to a patient in need of treatment for chronic inflammation or cancer metastasis.

8. The method according to claim 7, wherein the drug comprises one or more plant bodies selected from the group consisting of Japanese mugwort, dong quai and white dead-nettle, or their extracts.

9. The use of a drug that inhibits binding between EMMPRIN and S100A9 or S100A8/A9, for inhibition of chronic inflammation or cancer metastasis.

10. The use according to claim 9, wherein the drug comprises one or more plant bodies selected from the group consisting of Japanese mugwort, dong quai and white dead-nettle, or their extracts.


### [Sequence Listing]

SEQUENCE LISTING

<110>  SHISEIDO COMPANY, LTD.

<120>  Method for Screening of Anti-Inflammatory Agent as Indicator of
Presence or Absence of Inhibition of Binding Between Emmprin and S100A9

<130>  Y824-PCT

<150>  JP2010-174038
<151>  2010-08-02

<160>  15

<170>  PatentIn version 3.1

<210>  1
<211>  385
<212>  PRT
<213>  Homo sapiens

<400>  1

Met Ala Ala Ala Leu Phe Val Leu Leu Gly Phe Ala Leu Leu Gly Thr
1               5                   10                  15

His Gly Ala Ser Gly Ala Ala Gly Phe Val Gln Ala Pro Leu Ser Gln
            20                  25                  30

Gln Arg Trp Val Gly Gly Ser Val Glu Leu His Cys Glu Ala Val Gly
        35                  40                  45

Ser Pro Val Pro Glu Ile Gln Trp Trp Phe Glu Gly Gln Gly Pro Asn
    50                  55                  60

Asp Thr Cys Ser Gln Leu Trp Asp Gly Ala Arg Leu Asp Arg Val His
65                  70                  75                  80

Ile His Ala Thr Tyr His Gln His Ala Ala Ser Thr Ile Ser Ile Asp
                85                  90                  95

Thr Leu Val Glu Glu Asp Thr Gly Thr Tyr Glu Cys Arg Ala Ser Asn
            100                 105                 110

Asp Pro Asp Arg Asn His Leu Thr Arg Ala Pro Arg Val Lys Trp Val
        115                 120                 125

Arg Ala Gln Ala Val Val Leu Val Leu Glu Pro Gly Thr Val Phe Thr
    130                 135                 140

Thr Val Glu Asp Leu Gly Ser Lys Ile Leu Leu Thr Cys Ser Leu Asn
145                 150                 155                 160

Asp Ser Ala Thr Glu Val Thr Gly His Arg Trp Leu Lys Gly Gly Val
                165                 170                 175

Val Leu Lys Glu Asp Ala Leu Pro Gly Gln Lys Thr Glu Phe Lys Val
            180                 185                 190

Asp Ser Asp Asp Gln Trp Gly Glu Tyr Ser Cys Val Phe Leu Pro Glu
        195                 200                 205

Pro Met Gly Thr Ala Asn Ile Gln Leu His Gly Pro Pro Arg Val Lys
    210                 215                 220

Ala Val Lys Ser Ser Glu His Ile Asn Glu Gly Glu Thr Ala Met Leu
225                 230                 235                 240

Val Cys Lys Ser Glu Ser Val Pro Pro Val Thr Asp Trp Ala Trp Tyr
                245                 250                 255

Lys Ile Thr Asp Ser Glu Asp Lys Ala Leu Met Asn Gly Ser Glu Ser
            260                 265                 270

Arg Phe Phe Val Ser Ser Ser Gln Gly Arg Ser Glu Leu His Ile Glu
        275                 280                 285

Asn Leu Asn Met Glu Ala Asp Pro Gly Gln Tyr Arg Cys Asn Gly Thr
    290                 295                 300

Ser Ser Lys Gly Ser Asp Gln Ala Ile Ile Thr Leu Arg Val Arg Ser
305                 310                 315                 320

His Leu Ala Ala Leu Trp Pro Phe Leu Gly Ile Val Ala Glu Val Leu
                325                 330                 335

Val Leu Val Thr Ile Ile Phe Ile Tyr Glu Lys Arg Arg Lys Pro Glu
            340                 345                 350

Asp Val Leu Asp Asp Asp Asp Ala Gly Ser Ala Pro Leu Lys Ser Ser
        355                 360                 365

Gly Gln His Gln Asn Asp Lys Gly Lys Asn Val Arg Gln Arg Asn Ser
    370                 375                 380

Ser
385

<210>  2
<211>  21
<212>  DNA
<213>  Artificial

<220>
<223>  forward primer for IL-8

<400>  2
tcagagacag cagagcacac a                                                21


<210>  3
<211>  18
<212>  DNA
<213>  Artificial

<220>
<223>  reverse primer for IL-8

<400>  3
aatcaggaag gctgccaa                                                    18


<210>  4
<211>  20
<212>  DNA
<213>  Artificial

<220>
<223>  forward primer for TNFalfa

<400>  4
gacaagcctg tagcccatgt                                                  20


<210>  5
<211>  20
<212>  DNA
<213>  Artificial

<220>
<223>  reverse primer for TNFalfa

<400>  5
ttgatggcag agaggaggtt                                                  20


<210>  6
<211>  20
<212>  DNA
<213>  Artificial

<220>
<223>  forward primer for IL1F9

<400>  6
aggaagggcc gtctatcaat                                                  20


<210>  7
<211>  20
<212>  DNA
<213>  Artificial

<220>
<223>  reverse primer for IL1F9

<400>  7
aatgtgggct gttctccaac                                                  20

<210>  8
<211>  20
<212>  DNA
<213>  Artificial

<220>
<223>  forward primer for CXCL1

<400>  8
aaccgaagtc atagccacac                                                    20


<210>  9
<211>  22
<212>  DNA
<213>  Artificial

<220>
<223>  reverse primer for CXCL1

<400>  9
gttggatttg tcactgttca gc                                                 22


<210>  10
<211>  25
<212>  DNA
<213>  Artificial

<220>
<223>  forward primer for S100A8

<400>  10
gggcaagtcc gtgggcatca tgttg                                              25


<210>  11
<211>  30
<212>  DNA
<213>  Artificial

<220>
<223>  reverse primer for S100A8

<400>  11
ccagtaactc agctactctt tgggctttct                                         30


<210>  12
<211>  28
<212>  DNA
<213>  Artificial

<220>
<223>  forward primer for S100A9

<400>  12
gctcctcggc tttgggacag agtgcaag                                           28


<210>  13
<211>  30
<212>  DNA
<213>  Artificial

<220>
<223>  reverse primer for S100A9

<400>  13
gcatttgtgt ccaggtcctc catgatgtgt                                        30


<210>  14
<211>  20
<212>  DNA
<213>  Artificial

<220>
<223>  forward primer for GAPDH

<400>  14
gagtcaacgg atttggtcgt                                                   20


<210>  15
<211>  20
<212>  DNA
<213>  Artificial

<220>
<223>  reverse primer for GAPDH

<400>  15
tgggatttcc attgatgaca                                                   20